# EUROPEAN PATENT APPLICATION

(11) **EP 1 829 449 A1**
(43) Date of publication of application: **05.09.2007**
(21) Application number: 05809708.0
(22) Date of filing: 24.11.2005
(51) Int. Cl.: A01N 59/08, A01N 25/02, A61L 2/18

(54) **HYPOCHLOROUS ACID BASED STERILIZER COMPOSITION**

(30) Priority: 24.11.2004 JP 2004339194
(71) Applicant: Maruishi Pharmaceutical Co., Ltd., Osaka-shi, Osaka 541-0044 (JP); Tomita Pharmaceutical Co., Ltd., Naruto-shi, Tokushima 7710360 (JP)
(72) Inventor: IKEDA, Masahiro c/o MARUISHI PHARMACEUCTICAL CO., LTD.,, Osaka 538-0042 (JP); SOGA, Manabu Central Lab, MARUISHI PHARMACEUCTICAL CO., LTD.,, Osaka 538-0042 (JP); OKUNISHI, Junji c/o MARUISHI PHARMACEUCTICAL CO., LTD.,, Osaka 538-0042 (JP); OKAMOTO, Kazuki c/o MARUISHI PHARMACEUCTICAL CO., LTD.,, Osaka 538-0042 (JP); TAMAGAWA, Kazuhiko c/oTOMITA PHARMACEUTICAL C0.,LTD.,, Naruto-shi, Tokushima 771-0360 (JP); TAMURA, c/oTOMITA PHARMACEUTICAL C0.,LTD.,, Naruto-shi, Tokushima 771-0360 (JP)
(74) Representative: Escher, Thomas
(86) International application number: PCT/JP2005/021602
(87) International publication number: WO 2006/057311

(57) **Abstract**

An object of the present invention is to provide a hypochlorous acid based sterilizing composition which is less corrosive to metal and a method for sterilizing a subject using the same. Provided by the present application is an aqueous sterilizing composition which comprises:(a) hypochlorous acid or at least one component which can release hypochlorous acid in water, and (b) a buffering agent; wherein the concentration of available chlorine in the composition upon use is 0.0001-12%. An electrolyzed water may be used instead of component (a).
A method for sterilizing a subject using the aqueous sterilizing composition of the invention is also provided.

## Description

### Technical Field

The instant invention relates to a sterilizing composition. In more detail, the instant invention relates to a hypochlorous acid based sterilizing composition which can provide efficient sterilizing effect while causes suppressed metal corrosion, and a method for sterilizing a subject using said sterilizing composition.

### Background Art

Chlorine-based sterilizers such as sodium hypochlorite, calcium hypochlorite and sodium dichloroisocyanurate have been known as sterilizers which can be used in various environments. Those chlorine-based sterilizers are widely used in medical and public facilities as well as for general household activities. Sodium hypochlorite has been used for long time ago and can effectively kill common bacterium, bacterial spores, tubercle bacillus as well as viruses by oxidizing them. Sodium hypochlorite is degraded into water and sodium chloride and, therefore, is safe. Dilute aqueous sodium hypochlorite has been used in food industries as well as for sterilizing baby bottles. However, the conventional sodium hypochlorite sterilizing compositions are significantly inactivated by contamination from organic materials and therefore, concentrated sodium hypochlorite might be used for the sterilization of a portion to which blood and the like are adhered.

Chlorine-based sterilizers such as sodium hypochlorite and sodium dichloroisocyanurate have been used for long time because of their strong sterilizing property and safety. However, they cannot be used for sterilizing metal materials because they are corrosive to metal and therefore, have been used for sterilizing non-metal materials only. Patent literature 1 discloses to weaken the corrosive property of a sterilizing composition by adjusting its pH to 12.5-14.0 with an alkali metal hydroxide. The reference taught that the approach will not affect badly to the material to be sterilized.

In recent years, electrolyzed water, which is formed by electrolytic treatment of an aqueous solution of electrolytes including chlorine or aqueous hydrochloric acid has been attracted attention of the art because of its sterilizing property. However, it has similar problems as the conventional chlorine-based sterilizers that if the available chlorine concentration of the composition is not enough, the sterilizing property of the composition will be decreased significantly.

Patent Literature 1 Japanese Patent Laid Open H9-31494

### SUMMARY OF THE INVENTION

### PROBLEMS TO BE SOLVED BY THE INVENTION

An object of the instant invention is to provide a high concentration hypochlorous acid based sterilizing composition which is easy to use and cheap, and less corrosive to metal.

### MEANS TO SOLVE THE INVENTION

The instant inventors have found that the corrosive property of a hypochlorous acid based sterilizing composition can be suppressed by adding a buffering agent to the conventionally known hypochlorous acid based composition and completed the instant invention.

Namely, the instant application provides an aqueous sterilizing composition which comprises:
(a) hypochlorous acid or at least one component which can release hypochlorous acid in water, and
(b) a buffering agent,
wherein the concentration of available chlorine in the composition upon use is 0.0001-12%.

According to the present invention, examples of the buffering agent may preferably include organic acids, salts of organic acids, inorganic acids, salts of inorganic acids as well as pH buffering agents such as a mixture of an organic acid and a salt of organic acid, and a mixture of an inorganic acid and a salt of the inorganic acid. More preferably, organic acids, salts of organic acids, and mixture of an organic acid and a salt of the acid are used. When an organic acid, a salt of organic acid or a mixture of an organic acid and a salt of the organic acid is used as the buffering agent, the molar concentration of total acid group of the organic acid and the salt of the organic acid must be more than 5 times of the molar concentration of the available chlorine in the composition.

The instant invention also provides a method for sterilizing a subject having a part made of metal, which comprises: contacting said subject with the aqueous sterilizing composition of the present invention.

In this application, "aqueous sterilizing composition" represents a liquid composition comprising the components specified according to the invention, solid composition which will be dissolved in water upon use to provide the composition as well as a concentrated liquid composition which will be diluted with water upon use.

In the instant specification, "%" represents weight/volume % unless indicated otherwise.

This time, the inventors have found that the metal corrosive property of a conventionally known hypochlorous acid based sterilizing composition can be suppressed by adding a buffering agent to the composition without affecting antimicrobial spectrum of the composition and completed the instant invention. According to the present invention, a hypochlorous acid based sterilizing composition with a higher concentration of available chlorine and suppressed metal corrosive property is provided. The composition of the invention can be used safely for sterilizing various subjects including endoscopic instruments having metal parts is provided.
In addition, the sterilizing composition of the present invention exhibits similar anti-corrosive properties over a wide pH range including alkaline pH where compositions comprising an alkali metal salt of hypochlorous acid or chloroisocyanuric acid is used as well as an acidic pH where electrolyzed water formed by electrolytic treatment of an aqueous solution of electrolytes including chlorine or aqueous hydrochloric acid is used.

### BRIEF DESCRIPTION OF DRAWING(S)

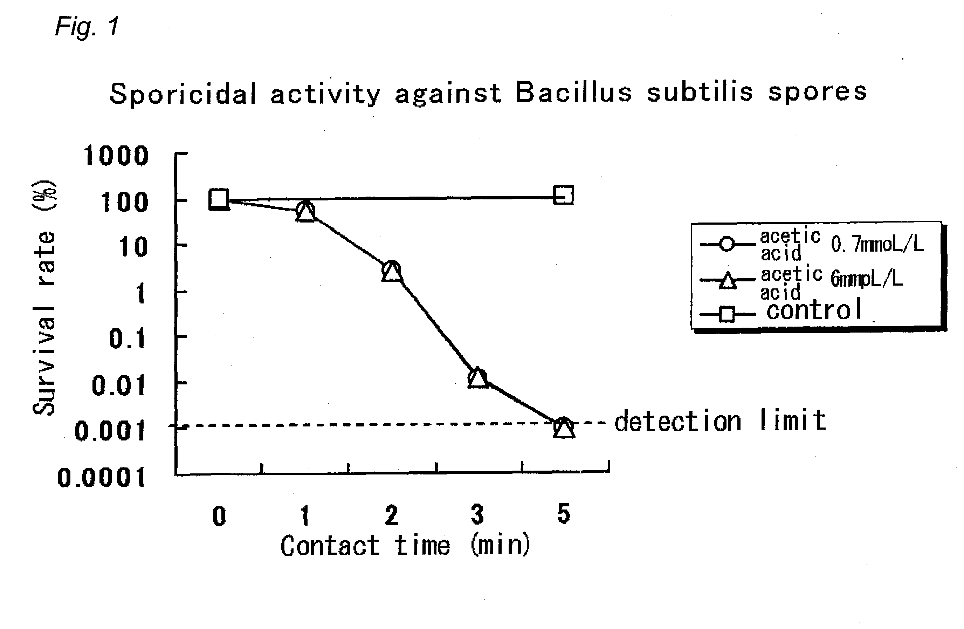

[Fig. 1] Graph comparing the antibacterial effect of sterilizing composition of the instant application and the conventionally known composition on spores.

### MOST PREFERRED EMBODIMENTS TO CONDUCT THE INVENTION

The component which can release hypochlorous acid in water used as component (a) of the present invention may be any material used in conventionally known chlorine based sterilizing compositions and examples of the same may include salts of hypochlorous acid, salts of dichloroisocyanuric acid, trichloroisocyanuric acid and chloramine-T. Examples of salts of hypochlorous acid may include alkali metal hypochlorite and especially, sodium hypochlorite and calcium hypochlorite. Especially preferable components may include sodium hypochlorite, calcium hypochlorite, sodium dichloroisocyanurate, trichloroisocyanuric acid and chloramine-t.

According to the present invention, the component (a) is contained in the composition so that the concentration of available chlorine in the composition upon use is 0.0001-12%. The concentration of the hypochlorous acid may be determined depending on the subject to be sterilized and the purpose of the sterilization. Typically, the concentration may be determined so that the concentration of available chlorine in the composition upon use is in the range of 0.0001-1%, especially, 0.001-0.05%.

In the present invention, electrolyzed water may be used as a composition comprising hypochlorous acid or a component which can release hypochlorous acid in water. The term "electrolyzed water" is generic term representing aqueous solution obtained by passing a weak direct-current thorough tap water or dilute aqueous sodium chloride. The electrolyzed water used in the present invention is strong electrolyzed water which has been used for hygiene control including those for washing and sterilizing. As strong electrolyzed water, strongly acidic electrolyzed water, weakly acidic electrolyzed water and electrolytic sodium hypochlorite solution are exemplified.

The most preferably used electrolyzed water is hypochlorous acid water which is yielded as anolyte water from 0.2% aqueous sodium chloride solution in a diaphragm type electrolytic cell or as electrolyzed solution from 2-6% aqueous hydrochloric acid solution in a undivided electrolytic cell.

In the instant invention, the electrolyzed water may be any of those containing available chlorin in a concentration comparative to the electrolyzed waters conventionally used for sterilization. Typically, the concentration of available chlorine in the composition upon use may be within the range of 0.0001-12%, preferably, 0.0001-1% and more preferably 0.001-0.05%. In order to adjust the concentration of available chlorine, the composition of the invention may be diluted with water or added with a component which can release hypochlrous acid in an aqueous solvent upon use.

As above disclosed, metal corrosive property of conventionally known hypochlorous acid based sterilizing compositions can be suppressed effectively by adding a buffering agent to the composition.

According to the present invention, examples of buffering agents may include inorganic acids, salts of inorganic acids, organic acids and salts of organic acids. In addition, pH buffering agents such as a mixture of an inorganic acid and a salt of the inorganic acid as well as a mixture of an organic acid and a salt of the organic acid are also preferably used.

Examples of inorganic acids may include phosphoric acid, boric acid and salts thereof.
Examples of organic acids may include mono carboxylic acids such as foric acid, acetic acid, propionic acid, butyric acid and acrylic acid, dicarboxylic acids such as oxialic acid, malonic acid, succinic acid, succinic acid, glutaric acid, adipic acid, maleic acid and fumaric acid, and salts thereof.

Examples of salts of inorganic or organic acids may include alkali metal salts of the acids and especially, sodium and potassium salts are preferable.

The buffering agents used in the present invention may preferably be pH buffering agents which is a mixture of an organic acid and a salt of the organic acid. More preferable buffering agents are acetic acid, an alkali metal acetate and a mixture of acetic acid and an alkali metal acetate.

According to the present invention, when acetic acid, an alkali metal acetate or a pH buffering agent consisting of acetic acid and an alkali metal acetate is used as buffering agent, the amount of the buffering agent should be adjusted so that the molar concentration of the acid group of the buffering agent is more than the molar concentration of the available chlorine in the composition. In this context, "molar concentration of the acid group" represents molar concentration of total carboxylic groups in the composition irrespective of the group is free or in the form of salt.
The molar concentration of the acid group must be more than 5 times that of the available chlorine and may be determined based on the time period required for the sterilizing step as well as the type of metal to be sterilized. When the aqueous sterilizing composition comprises 30ppm of hypochlorous acid, for example, the concentration of the acid group of the buffering agent may preferably be from 3mmol/L and up to the highest concentration at which the salt is not precipitated, and especially 5-100 mmol/L.

The pH of the sterilizing composition of the present invention may be adjusted so that the pH upon use is pH 2-13, preferably, pH 2-11 and mopre preferably pH 2-7.

The sterilizing composition of the present invention may also preferably be used under an alkalin condition. The alkaline condition may be pH 7-13.

The pH of the sterilizing composition of the invention may be adjusted by adding a conventionally known pH controlling agent such as a strong alkaline agent like sodium hydroxide to the composition comprising the certine amount of the buffering agent.

The sterilizing composition of the present invention may be provided as stock solution containing equal to or less than 10%, preferably 1-6% of hypochlorous acid. The stock solution may be adjusted to pH 9-13, and preferably, to pH 10-13.
The stock solution of the sterilizing composition of the present invention may contain the buffering agent. Alternatively, the stock solution containing no buffering agent may be admixed with the buffering agent simultaneously with diluting the same upon use. The stock solution may be diluted with purified water or with tap water, and tap water also can provide the composition for use with enough property.

In case a pH buffering agent consisting of an acid and a salt thereof is used as the buffering agent, the pH of the composition may be adjusted by said pH buffering agent.

The sterilizing composition of the invention may further comporise a surfactant. The ability of the composition to wash the subject will be enhanced by admixining a surfactant. Examples of surfactants may inglude anionic surfactants like alkyl sulfates such as sodium polyoxyethylene alkylether sulfate, sodium polyoxyethylene laurylether sulfate, sodium polyoxyethylene cetylether sulfate and sodium polyoxyetylene stearylether sulfate; amphpteric surfactants including alkylamine oxides such as lauryldimethylamine oxide, dihydroxyethyl lauril amine oxide and dimethyl oleylamine oxide, alkyl betaines such as coconut oil alkyl betaine and betaine lauryldimethyl acetate; and pluronic type nonionic surfactants like polypropyleneglycole ethylene oxide adduct such as polyoxyethylene polyoxypropylene block polymer.
One of the most preferable surfactants is salt of alkylether sulfate.

The amount of the surfactant in the sterilizing composition of the invention is not limited and may be the amount comparative to those contained in conventional sterilizing compositions. In general, 0.001-10% and typically, about 1% of surfactant may be contained in the sterilizing composition of the invention upon use.

The sterilizing composition of the invention may further comprise any other components that are usually contained in conventional sterilizing compositions so long as they do not contradict the object of the present invention.

According to the present invention, sterilizing composition may be used in a manner similar to the conventionally known hypochlorous acid based sterilizing compositions containing no acetic acid or a salt thereof. When a sterilizing composition containing 30ppm of sodium hypochlorite is used, for example, the composition may be contacted with a subject to be sterilized for equal to or more than 30 seconds to kill viable bacterium and equal to or more than 5 minutes to kill spores effectively.

Although the steriliz ing composition of the present invention may be used for sterilizing any materials which have been sterilized to date with conventional chlorine-based sterilizing composition, the composition of the invention is especially useful for sterilizing a subject having a part made of metal because the composition of the present invention is less corrosive to metal. The subject having a part made of metal to be sterilized with the composition of the invention is not limited and may be any equipments which are required to be sterilized, for example, medical equipments such as endoscopes or cooking equipments, various devices and equipments used in pharmaceutical or food industries. When a subject such as endoscope made of a specific type of metal or adhesive is sterilized, the sterilizing condition including the concentration of the sterilizing composition and the time period must be controlled precisely and therefore, the sterilizing process may preferably be carried out by using a conventionally used sterilizing device.

### EXAMPLES

The present invention will be further illustrated by referring the examples below.

### Example 1

Sodium hypochlorite was dissolved in purified water to give 30ppm (0.4mM) solution. Acetic acid was added thereto so that the concentration of the same is 6mmol/L. Thus obtained solution was adjusted to pH 5 with 1N sodium hydroxide to give sterilizing composition of Example 1.

### Comparative Example 1

Sodium hypochlorite was dissolved in purified water to give 30ppm solution. Thus obtained solution was adjusted to pH 5 with acetic acid to give the sterilizing composition of Comparative Example 1. The acetic acid concentration in the composition was 0.7mmol/L.
Corrosive property of the composition to metal was determined using test pieces made of iron, copper, steel, and stainless steel SUS420J2 and SUS304. Each of thus obtained sterilizing compositions of Example 1 and Comparative Example 1 was poured in a vessel and test pieces were immersed therein. The vessels with the test pieces were stood at the room temperature (about 20°C) and the time-dependent development of corrosion on the surface of the strips were visually observed. Strips made of iron, copper and steel were observed for one hour and those made of stainless steel were for two hours. Results are shown in Table 1.

**Table 1**

| | | |
|---|---|---|
| 30 ppm aqueous sodium hypochlorate | | |

| test pieces | comparative example 1 acetic acid 0.7mmol/L | Example 1 acetic acid 6mmol/L |
|---|---|---|
| iron | corrosion was observed between 30 min and 1 hour | no corrosion was observed until 1 hour |
| copper | corrosion was observed between 5 and 10 minutes | corrosion was observed between 30 min and 1 hour |
| steel | corrosion was observed between 0 and 5 minutes | corrosion was observed between 30 min and 1 hour |
| SUS420J2 | corrosion was observed between 1 and 2 hours | no corrosion was observed until 2 hours |
| SUS304 | no corrosion was observed until 2 hours | no corrosion was observed until 2 hours |

### Examples 2-7 and Comparative Examples 3, 4

Sodium hypochlorite was dissolved in purified water to give solutions containing 30ppm and 60ppm of sodium hypochlorite respectively. Acetic acid was added to each solution to give solutions containing 5mmol/L, 10mmol/L and 15mmol/L of acetic acid and 30ppm of sodium hypochlorite and those containing 10mmol/L, 20mmol/L and 30mmol/L of acetic acid and 60ppm of sodium hypochlorite. Each of the solutions was adjusted so that the final pH is 5 with sodium hydroxide to give sterilizing composition of the examples.

As for comparative example, sodium hypochlorite was dissolved in purified water to give 30ppm and 60ppm solutions, acetic acid was added thereto and the pH of the solutions were adjusted to 5. The acetic acid concentrations were adjusted to 0.7mmol/L for 30ppm hypochlorite solution and 1.2mmol/L for 60 ppm hypochlorite solution.
Test piece made of stainless steel SUS420J2 was immersed in the respective sterilizer solutions and kept stood at the room temperature (about 20°C) for 24 hours. Time dependent development of corrosion on the surface of the test piece was observed visually. Results are shown in Table 2.

**Table 2**

| | sodium hypochlorite | acetic Corrosion on test piece acid SUS420J2 (mmol/L) | |
|---|---|---|---|
| Comp. Ex. 2 | 30ppm | 0.7 | corrosion was observed between 1 and 2 hours |
| Example2 | | 5 | no corrosion was observed until 24 hours |
| Example3 | | 10 | no corrosion was observed until 24 hours |
| Example4 | | 15 | no corrosion was observed until 24 hours |
| Comp. Ex. 3 | 60ppm | 1.2 | corrosion was observed between 10 and 15 minutes |
| Example5 | | 10 | corrosion was observed between 1 and 2 hours |
| Example6 | | 20 | no corrosion was observed until 24 hours |
| Example7 | | 30 | no corrosion was observed until 24 hours |

In comparative example 2, corrosion was observed at an early stage while in examples 2-4, no corrosion was observed within 24 hours of immersion. Those results support that addition of acetic acid suppress the metal corrosion. In general, the higher the concentration of sodium hypochlorite, the earlier development of metal corrosion could be observed. The time until corrosion was observed in Examples 5-7, however, was significantly prolonged than that in Comparative Example 3. The sterilizing composition of Examples 5-7 can also be available in real usage.

### Example 8

A solution of sodium hypochlorite 0.03% (300ppm) and acetic acid 3.36% (0.56M) in purified water was prepared and adjusted to pH 5 with 1N sodium hydrochloride to give sterilizing composition of Example 8.
A test piece made of stainless steel SUS420J2 was immersed in the composition and kept stood at the room temperature (about 20°C) for 24 hours. No corrosion was observed until 24 hours.
It can be concluded that the sterilizing composition of the present invention comprising a relatively high concentration of sodium hypochlorite has enough capability to prevent corrosion damage.

After the sterilizing composition shown as example 3 (30ppm of sodium hypochlorite and 10mmol/L of acetic acid, which was adjusted to pH 5) was contacted with spores of *Bacillus subtilis,* viable cell counts were determined over time. Survival rate was calculated as percent of the initial count (0 minute). As a positive control, the composition of comparative example 2 consists of sodium hypochlorite 30ppm, acetic acid 0.7mmol/L which was adjusted to pH 5, and as a negative control, purified water were used. The results are shown in Figure 1.
The sterilizing composition of example 3 of the invention as well as positive control showed the decrease of the cell viability to below the level of detection, i.e. 99.999% sporicidal activity was confirmed within 5 minutes.

### Stability Test

The sterilizing compositions of examples 1 and 5, and comparative example 1 were prepared and kept stood at the room temperature. The concentration of sodium hypochlorite was determined at 7th and 14th days after the preparation by the iodometric titration method. The residual sodium hypochlorite was calculated as percent (%) of the initial concentration. Results are shown in Table 3.

**Table 3**

| | sodium hypochlorite | acetic acid (mmol/L) | Residual Ratio (%) | |
|---|---|---|---|---|
| | | | 7th day | 14th day |
| Com. Ex. 1 | 30ppm | 0.7 | 98.1 | 94.7 |
| Ex. 1 | 30ppm | 6 | 98.4 | 95.4 |
| Ex. 5 | 60ppm | 10 | 97.0 | 96.1 |

The sterilizing composition of the present invention exhibited a stability equivalent to that of comparative example, a conventional sterilizing composition.

Metal parts of endoscope (Fuji Photo Optical Co., Ltd.) were immersed in each sterilizing composition of Examples 1-7 and kept for 5 days at the room temperature. No metal discoloration nor metal corrosion were observed in all compositions.

### Example 9 and Comparative Example 4 pH stability test

Sodium hypochlorite was dissolved in purified water to give 500ppm solutions. Potassium acetate 0.7M was added to a half of the solution to give sterilizing composition of Example 9 and the remaining solution was used as composition of comparative example 4. The pH of thus obtained compositions were 10.6 and 10.3 respectively.
Thus obtained sterilizing composition was poured in a vessel, a test piece made of SUS420J2 was soaked therein and development of corrosion on the surface of the piece was observed. No corrosion was observed on the surface of the test piece immersed in composition of example 9 which comprises potassium acetate until 24 hours, while corrosion was observed between 30 minutes-1hour in composition of comparative example 4. Results are shown in Table 4.

### Examples 10 and 11

Sodium hypochlorite was dissolved in purified water to give two containers of 30ppm solution. Acetic acid 3.6M was added to the one container to give sterilizing composition of Example 10 and acetic acid 71.6mM was added the other to give sterilizing composition of Example 11. The pH of thus obtained compositions were adjusted to 2.0 and 3.0 respectively.
Thus obtained sterilizing composition was poured in a vessel, a test piece made of SUS420J2 was immersed therein and development of corrosion on the surface of the strip was observed. No corrosion was observed on the surface of the test piece immersed in composition of example 10 or example 11 until 24 hours. Results are shown in Table 4.

**Table 4**

| | Sodium hypochlorite | buffering agent | pH | corrosion on test piece SUS420J2 |
|---|---|---|---|---|
| Ex. 9 | 500 ppm | potassium acetate 0.7M | 10.6 | no corrosion was observed until 24 hours corrosion was observed between 30 min and 1 hour |
| Comp. Ex. 4 | | none | 10.3 | |
| Ex. 10 | 30 ppm | acetic acid 3.6M | 2.0 | no corrosion was observed until 24 hours |
| Ex. 11 | | acetic acid 71.6mM | 3.0 | no corrosion was observed until 24 hours |

## Claims

1. An aqueous sterilizing composition which comprises:
(a) hypochlorous acid or at least one component which can release hypochlorous acid in water, and
(b) a buffering agent,
wherein the concentration of available chlorine in the composition upon use is 0.0001-12%.

2. The composition according to Claim 1, wherein the buffering agent is an organic acid, a salt of an organic acid, or a mixture of an organic acid and a salt of the organic acid.

3. The composition according to Claim 2, wherein the buffering agent is acetic acid, a salt of acetic acid, or a mixture of acetic acid and a salt thereof.

4. The composition according to Claim 2 or 3, wherein the molar concentration of the acid group from the organic acid and the salt of organic acid is more than 5 times of the molar concentration of available chlorine.

5. The composition according to any one of Claims 1-4,
wherein the at least one component which can release hypochlorous acid in water is selected from the group consisting of an alkali metal hypochlorite, calcium hypochlorite, dichloroisocyanuric acid, trichloroisocyanuric acid and chloramine-T.

6. The composition according to any one of Claims 1-4,
wherein the component which can release hypochlorous acid in water is electrolyzed water.

7. The composition according to Claim 5, wherein the electrolyzed water is acidic electrolyzed water obtained by electrolyzing an aqueous solution selected from the group consisting of a solution containing equal to or less than 0.2% sodium chloride and a solution containing 2-6% hydrochloric acid.

8. The composition according to any one of Claims 1-6,
wherein the concentration of available chlorine in the composition upon use is 0.001-0.05%.

9. The composition according to any one of Claims 1-7, further comprising a surfactant.

10. The composition according to Claim 9, wherein the surfactant is selected from the group consisting of anionic surfactants, amphoteric surfactants and pluronic type nonionic surfactants.

11. The composition according to Claim 10, wherein the surfactant is selected from the group consisting of alkylether sulfates, alkylamine oxides, alkyl betaines and polypropyleneglycole ethylene oxide adduct.

12. The composition according to Claim 11, wherein the surfactant is an alkylether sulfate.

13. The composition according to Claim 12, wherein the alkylether sulfate is selected from the group consisting of sodium polyoxyethylene alkylether sulfate, sodium polyoxyethylene laurylether sulfate, sodium polyoxyethylene cetylether sulfate and sodium polyoxyetylene stearylether sulfate.

14. The composition according to any one of Claims 1-13,
wherein the pH of the composition upon use is in the range of pH 2-13.

15. The composition according to Claim 14, wherein the pH of the composition upon use is in the range of pH 2-7.

16. The composition according to Claim 14, wherein the pH of the composition upon use is in the range of pH 7-13.

17. The composition according to any one of Claims 1-16, which is used for sterilizing a subject having a part made of metal.

18. The composition according to Claim 17, wherein the subject having a part made of metal is a medical equipment.

19. The composition according to Claim 18, wherein the medical equipment is a endoscope.

20. A method for sterilizing a subject having a part made of metal, which comprises contacting the composition according to any one of Claims 1-19 with the subject.

21. The method of Claim 20, wherein the subject having a part made of metal is a medical equipment.

22. The method of Claim 21, wherein the subject having a part made of metal is endoscope.
